# EUROPEAN PATENT SPECIFICATION

(11) **EP 2 934 333 B1**
(45) Date of publication and mention of the grant of the patent: **15.05.2019**
(21) Application number: 13864905.8
(22) Date of filing: 18.12.2013
(51) Int. Cl.: G01S 15/89, A61B 8/00, A61B 8/08

(54) **METHOD FOR MULTI-FREQUENCY IMAGING USING HIGH-BANDWIDTH TRANSDUCER OUTPUTS**
VERFAHREN FÜR MEHRFREQUENZ-BILDGEBUNG MITTELS WANDLERAUSGÄNGEN VON HOHER BREITE
PROCÉDÉ POUR IMAGERIE MULTIFRÉQUENCE UTILISANT DES SORTIES DE TRANSDUCTEUR DE LARGEUR DE BANDE ÉLEVÉE

(30) Priority: 21.12.2012 US 201261740822 P
(43) Date of publication of application: 28.10.2015
(73) Proprietor: Volcano Corporation, San Diego, CA 92130 (US)
(72) Inventor: RICE, Cheryl, D., San Diego, CA 92129 (US)
(74) Representative: de Haan, Poul Erik
(86) International application number: PCT/US2013/076195
(87) International publication number: WO 2014/100217

(56) References cited:
- WO-A1-2012/032849
- WO-A1-2012/144243
- WO-A2-03/079038
- US-A1- 2002 040 188
- US-A1- 2007 208 253
- US-A1- 2008 114 245
- US-A1- 2008 200 815
- US-A1- 2010 156 243
- US-A1- 2010 174 190
- US-A1- 2010 241 005
- US-A1- 2011 160 586
- US-A1- 2012 197 113
- US-B1- 6 248 073

## Description

### Field of the Invention

The present disclosure relates generally to intravascular ultrasound (IVUS) imaging inside the living body and, in particular, to an IVUS imaging catheter that produces high resolution intravascular multi-frequency imaging using high bandwidth transducer outputs.

### Description of Related Art

Intravascular ultrasound (IVUS) imaging is widely used in interventional cardiology as a diagnostic tool for a diseased vessel, such as an artery, within the human body to determine the need for treatment, to guide the intervention, and/or to assess its effectiveness. IVUS imaging uses ultrasound echoes to create an image of the vessel of interest. The ultrasound waves pass easily through most tissues and blood, but they are partially reflected from discontinuities arising from tissue structures (such as the various layers of the vessel wall), red blood cells, and other features of interest. The IVUS imaging system, which is connected to an IVUS catheter by way of a patient interface module (PIM), processes the received ultrasound echoes to produce a cross-sectional image of the vessel where the catheter is placed.

Current IVUS solutions do not provide the resolution capable of differentiating structures without significant training in image interpretation. Structures requiring clearer images might include plaque burden, stent apposition, lipid pool identification, thrombus, and stent endothelization. While Optical Coherence Tomography (OCT) devices offer improved resolution, they require flushing to produce the image, and due to limitations of light penetration they do not allow for visualization of the vessel morphology beyond the surface of the vessel. Furthermore, state-of-the art systems may obtain improved image resolution by operating at higher frequencies, but at the cost of reduced tissue penetration. Likewise, state-of-the-art systems that operate at lower frequencies provide deeper tissue penetration but at a lower axial resolution.

Document US 2012197113 A1 describes an apparatus for electrically addressing multiple ultrasonic transducers that are connected to a common electrical channel and housed within an imaging probe. An imaging probe may comprise an imaging ultrasonic transducer and a moveable element for controlling the direction of an emitted imaging beam, and an angle sensing ultrasonic transducer, where the angle sensing ultrasonic transducer is configured for determining the direction of an ultrasonic imaging beam.

Document US 2008114245 A1 describes a device for conducting ultrasound interrogation of a medium. The method includes transmitting a non-beamformed or beamformed ultrasound wave into the medium, receiving more than one echoed ultrasound wave from the medium, and converting the received echoed ultrasound wave into digital data.

While existing IVUS catheters deliver useful diagnostic information, there is a need for enhanced image quality to provide more valuable insight into the vessel condition. For further improvement in image quality in rotational IVUS, it is desirable to use a transducer with broader bandwidth to incorporate multiple frequencies in the image creation process.

What is needed is a method for multi-frequency intravascular imaging to assess lesions, characterize vessels or to monitor other structures within a patient's body.

### Summary

The invention is defined by claim 1 and wherein an intravascular ultrasound imaging system for imaging a volume within a patient comprises:
an interface module comprising a processing circuit and an IVUS catheter comprising a plurality of piezo-electric transducer components at the distal end, said plurality having a first transducer component for generating an ultrasonic signal at a first selected frequency and a second transducer component for receiving an ultrasonic echo at a second selected frequency, wherein the second frequency is a harmonic of the first frequency; and
wherein the second transducer component has a response band including a portion of a reception band; and
wherein the IVUS catheter is configured to direct the ultrasonic signal on a spot in the volume within the patient and scan the spot in a predetermined pattern about the wall of the volume within the patient; and
wherein the second transducer component is configured to convert the received ultrasonic echo into a voltage signal including the second selected frequency; and
wherein the interface module comprises a receive amplifier having filters with bandwidths producing said reception band selected according to the said response band and configured to amplify the voltage signal with a processing circuit; and
wherein the IVUS imaging system is configured to provide an image of the volume within the patient from the amplified voltage signal.

Embodiments of the present invention will be described in further detail below with reference to the following drawings.

### Brief Description of the Drawings

FIG. 1A is a schematic illustration of an intravascular ultrasound (IVUS) imaging system.
FIG. 1B is a cross-sectional side view of a distal portion of a catheter used in an IVUS imaging system.
FIG. 2 is a block diagram of a Patient Interface Module (PIM) for use in an IVUS imaging system.
FIG. 3A is a partial illustration of a distal end of a catheter for multi-frequency imaging.
FIG. 3B is a partial illustration of a distal end of a catheter for multi-frequency imaging, according to some embodiments.
FIG. 4 is a partial schematic illustration of a transducer voltage.
FIG. 5 is a partial schematic illustration of multi-frequency voltage pulses for a transducer component.
FIG. 6 is a partial schematic illustration of a transmission band, a reception band, and a response band in a multi-frequency IVUS imaging system, according to some embodiments.
FIG. 7 is a partial schematic illustration of a signal processing strategy for selecting harmonic components of a signal, according to some embodiments.
FIG. 8 is a flow chart of a method for multi-frequency imaging.
FIG. 9 is a flow chart of a method for multi-frequency imaging.

In the figures, elements having the same reference number have the same or similar functions.

### Detailed Description

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

In embodiments of an IVUS catheter disclosed herein, an ultrasound transducer assembly is located at the tip of a flexible driveshaft that spins inside a plastic sheath inserted into the vessel of interest. The transducer assembly includes components oriented such that an ultrasound beam produced by the component propagates generally perpendicular to the axis of the catheter. A fluid-filled sheath protects the vessel tissue from the spinning transducer and driveshaft while permitting ultrasound signals to freely propagate from the transducer into the tissue and back. As the driveshaft rotates (typically at 30 revolutions per second), the transducer is periodically excited with a high voltage pulse to emit a short burst of ultrasound. The same transducer then listens for the returning echoes reflected from various tissue structures, and the IVUS imaging system assembles a two dimensional display of the vessel cross-section from a sequence of several hundred of these pulse/acquisition cycles occurring during a single revolution of the transducer.

In a rotational IVUS catheter, the ultrasound transducer may be a piezoelectric ceramic element with low electrical impedance capable of directly driving an electrical cable connecting the transducer to the imaging system hardware. In this case, a single pair of electrical leads (or coaxial cable) can be used to carry the transmit pulse from the system to the transducer and to carry the received echo signals from the transducer back to the imaging system by way of a patient interface module ("PIM") where echo signals can be assembled into an image. In embodiments where the catheter driveshaft and transducer are spinning (in order to scan a cross-section of the artery) and the imaging system hardware is stationary, an electromechanical interface couples the electrical signal to a rotating junction. In rotational IVUS imaging systems, this may be achieved by using a rotary transformer, slip rings, rotary capacitors, etc.

In some embodiments, an IVUS catheter may include a plurality of transducer components in a static configuration, forming a phased-array transducer assembly.

Reference will now be made to a particular embodiments of the concepts incorporated into an intravascular ultrasound system. However, the illustrated embodiments and uses thereof are provided as examples only. Without limitation on other systems and uses, such as but without limitation, imaging within any vessel, artery, vein, lumen, passage, tissue or organ within the body. While the following embodiments may refer to a blood vessel and a blood vessel wall for illustrative purposes, any other tissue structure may be envisioned to be imaged according to methods disclosed herein. More generally, any volume within a patient's body may be imaged according to embodiments disclosed herein, the volume including vessels, cavities, lumens, and any other tissue structures, as one of ordinary skill may recognize.

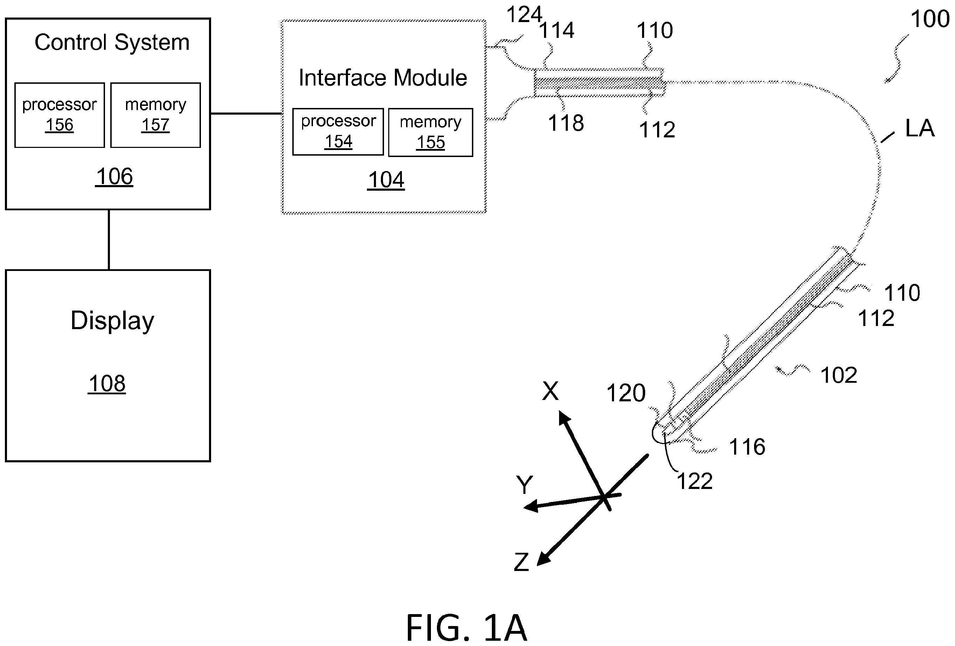

FIG. 1A is a schematic illustration of an intravascular ultrasound (IVUS) imaging system 100. IVUS imaging system 100 includes an IVUS catheter 102 coupled by a patient interface module (PIM) 104 to an IVUS control system 106. Control system 106 is coupled to a monitor 108 that displays an IVUS image (such as an image generated by IVUS system 100).

In some embodiments, catheter 102 is a rotational IVUS catheter, which may be similar to a Revolution® Rotational IVUS Imaging Catheter available from Volcano Corporation and/or rotational IVUS catheters disclosed in U.S. Patent No. 5,243,988 and U.S. Patent No. 5,546,948. In some embodiments, catheter 102 maybe a stationary component.

Catheter 102 includes an elongated, flexible catheter sheath 110 (having a proximal end portion 114 and a distal end portion 116) shaped and configured for insertion into a lumen of a blood vessel (not shown). In some embodiments, IVUS system 100 may be used for neurological evaluations in blood vessels in the brain, and for renal denervation in blood vessels in the kidney. A longitudinal axis LA of catheter 102 extends between the proximal end portion 114 and the distal end portion 116. Catheter 102 is flexible such that it can adapt to the curvature of the blood vessel during use. In that regard, the curved configuration illustrated in FIG. 1A is for exemplary purposes and in no way limits the manner in which catheter 102 may curve in other embodiments. Generally, catheter 102 may be configured to take on any desired straight or arcuate profile when in use.

In some embodiments an imaging core 112 extends within sheath 110. Accordingly, in some embodiments imaging core 112 may be rotated while sheath 110 remains stationary. Imaging core 112 has a proximal end portion 118 disposed within the proximal end portion 114 of sheath 110 and a distal end portion 120 disposed within the distal end portion 116 of sheath 110. The distal end portion 116 of sheath 110 and the distal end portion 120 of imaging core 112 are inserted into the vessel of interest during operation of the IVUS imaging system 100. The usable length of catheter 102 (for example, the portion that can be inserted into a patient, specifically the vessel of interest) can be any suitable length and can be varied depending upon the application. Proximal end portion 114 of sheath 110 and proximal end portion 118 of imaging core 112 are connected to PIM 104. Proximal end portions 114, 118 are fitted with a catheter hub 124 that is removably connected to PIM 104. Catheter hub 124 facilitates and supports a rotational interface that provides electrical and mechanical coupling between catheter 102 and PIM 104.

Distal end portion 120 of imaging core 112 includes a transducer assembly 122. In some embodiments, transducer assembly 122 is configured to be rotated (either by use of a motor or other rotary device, or manually by hand) to obtain images of the vessel. Transducer assembly 122 can be of any suitable type for visualizing a vessel and, in particular, a stenosis in a vessel. In Fig. 1A, transducer assembly 122 includes a piezoelectric micro-machined ultrasonic transducer ("PMUT") and associated circuitry, such as an application-specific integrated circuit (ASIC). An exemplary PMUT used in IVUS catheters may include a polymer piezoelectric membrane, such as that disclosed in U.S. Patent No. 6,641,540. The PMUT may provide greater than 100% bandwidth for optimum resolution in a radial direction, and a spherically-focused aperture for optimum azimuthal and elevation resolution. Thus, transducer assembly 122 may provide a focused ultrasonic beam having a spot size of about 50 µm or less.

In some embodiments transducer assembly 122 may include a plurality of stationary components disposed around the circumference of distal end 120 of catheter 102. In such configuration, the components in transducer 122 may be piezo-electric elements distributed to form a phased-array configuration. The piezo-electric elements may be ceramic-based or polymer-based. Furthermore, in some embodiments the plurality of stationary components in transducer 122 may be configured to produce a focused acoustic impulse. In such embodiments, the stationary components produce an acoustic impulse according to a pre-selected excitation phase for each of the components.

Transducer assembly 122 may also include a housing having the PMUT and associated circuitry disposed therein. In some embodiments the housing has an opening that ultrasound signals generated by the PMUT transducer travel through. In an alternative embodiment, the transducer assembly 122 includes an ultrasound transducer array (for example, arrays having 16, 32, 64, or 128 components are utilized in some embodiments).

In some embodiments, a rotation of imaging core 112 within sheath 110 is controlled by PIM 104. For example, PIM 104 provides user interface controls that can be manipulated by a user. In some embodiments PIM 104 receives, analyzes, and displays information received through imaging core 112. It will be appreciated that any suitable functionality, controls, information processing and analysis, and display can be incorporated into PIM 104. Thus, PIM 104 may include a processor circuit 154 and a memory circuit 155 to execute operations on catheter 102 and receive, process, and store data from catheter 102. In some embodiments PIM 104 receives data associated to ultrasound signals (echoes) detected by imaging core 112. PIM 104 processes the data and forwards the processed echo data to control system 106. Control system 106 may include a processor circuit 156 and a memory circuit 157 to execute operations on catheter 102 and receive, process, and store data from catheter 102. In some embodiments, PIM 104 performs preliminary processing of the echo data prior to transmitting the echo data to control system 106. PIM 104 may perform amplification, filtering, and/or aggregating of the echo data, using processor circuit 154 and memory circuit 155. PIM 104 can also supply high- and low-voltage DC power to support operation of catheter 102 including circuitry within transducer assembly 122.

In some embodiments, wires associated with IVUS imaging system 100 extend from control system 106 to PIM 104. Thus, signals from control system 106 can be communicated to PIM 104 and/or vice versa. In some embodiments, control system 106 communicates wirelessly with PIM 104. Similarly, it is understood that, in some embodiments, wires associated with IVUS imaging system 100 extend from control system 106 to monitor 108 such that signals from control system 106 can be communicated to monitor 108 and/or vice versa. In some embodiments, control system 106 communicates wirelessly with monitor 108.

Piezoelectric micro-machined ultrasound transducers (PMUTs) fabricated using a polymer piezoelectric material for use in transducer assembly 122, such as disclosed in U.S. Patent 6,641,540, offer greater than 100% bandwidth for optimum resolution in the radial direction, and a spherically-focused aperture for optimum azimuthal and elevation resolution.

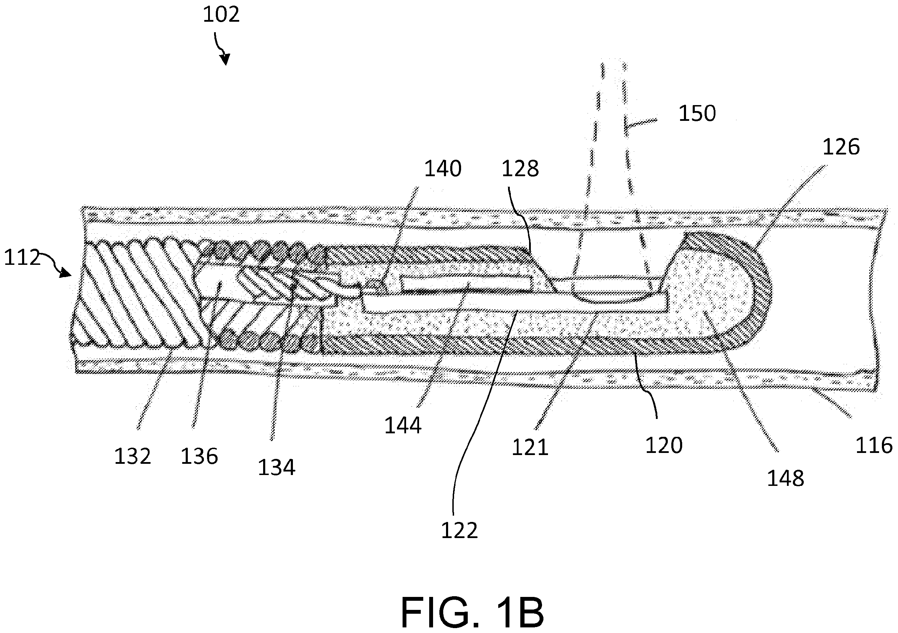

FIG. 1A illustrates a 3-dimensional (3D) Cartesian coordinate system XYZ oriented such that the Z-axis is aligned with the LA. In further descriptions of embodiments disclosed herein, a reference to a Cartesian plane or coordinate may be made in relation to FIG. 1. One of ordinary skill will recognize that the particular choice of coordinate axes in FIG. 1A is not limiting of embodiments as disclosed herein. The choice of coordinate axes is done for illustration purposes only.

FIG. 1B is a cross-sectional side view of a distal portion of a catheter used in an IVUS imaging system. In particular, Fig. 1B shows an expanded view of aspects of the distal portion of imaging core 112. In Fig. 1B, imaging core 112 is terminated at its distal tip by a housing 126 having a rounded nose and a cutout 128 for the ultrasound beam 150 to emerge from the housing. In some embodiments, a flexible driveshaft 132 of imaging core 112 is composed of two or more layers of counter wound stainless steel wires, welded, or otherwise secured to housing 126 such that rotation of the flexible driveshaft also imparts rotation to housing 126. In Fig. 1B, a PMUT MEMS transducer layer 121 includes a spherically focused portion facing cutout 128. In some embodiments, transducer assembly 122 may include application-specific integrated circuit (ASIC) 144 within distal portion 120 of imaging core 112. ASIC 144 is electrically coupled to transducer layer 221 through two or more connections.

In some embodiments of the present disclosure ASIC 144 may include an amplifier, a transmitter, and a protection circuit associated with PMUT MEMS layer 121. In some embodiments, ASIC 144 is flip-chip mounted to a substrate of the PMUT MEMS layer 121 using anisotropic conductive adhesive or suitable alternative chip-to-chip bonding method. When assembled together PMUT MEMS layer 121 and ASIC 144 form an ASIC/MEMS hybrid transducer assembly 122 mounted within housing 126. An electrical cable 134 with optional shield 136 may be attached to transducer assembly 122 with solder 140. Electrical cable 134 may extend through an inner lumen of the flexible driveshaft 132 to proximal end 118 of imaging core 112. In proximal end 118, cable 134 is terminated to an electrical connector portion of a rotational interface coupling catheter 102 to PIM 104 (cf. FIG. 1A). In Fig. 1A, transducer assembly 122 is secured in place relative to the housing 126 by an epoxy 148 or other bonding agent. Epoxy 148 may serve as an acoustic backing material to absorb acoustic reverberations propagating within housing 126 and as a strain relief for the electrical cable 134 where it is soldered to transducer assembly 122.

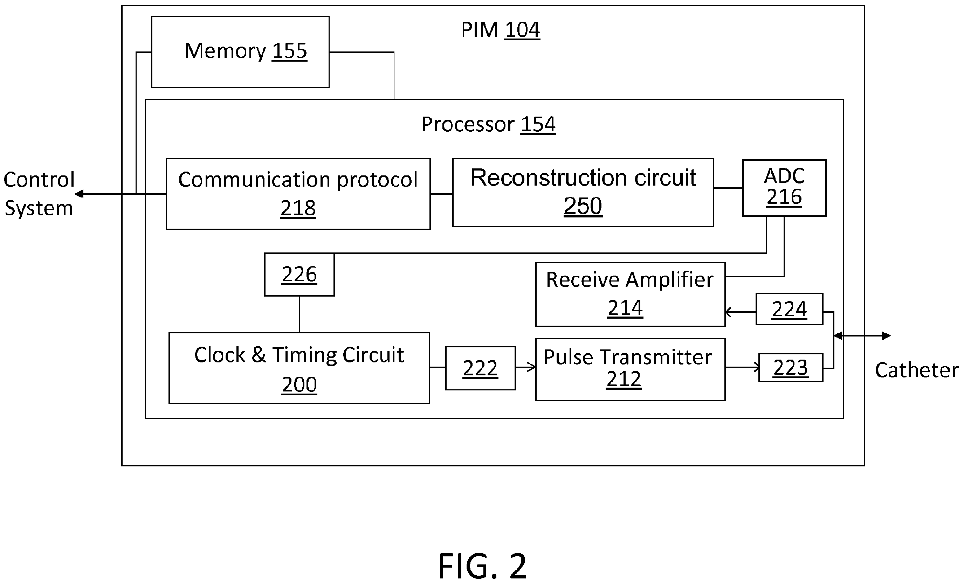

FIG. 2 is a block diagram of a Patient Interface Module (PIM) 104 for use in an IVUS imaging system. PIM 104 includes processor circuit 154 and memory circuit 155, described in detail above in relation to FIG. 1. PIM 104 provides a control signal 223 to a catheter, and receives data 224 from the catheter (e.g., catheter 102, FIG. 1). Control signal 223 may include a sequence of voltage pulses creating an acoustic impulse from a transducer assembly (e.g., transducer assembly 122). Control signal 223 may be generated in a pulse transmitter 212 included in processor circuit 154. Each pulse from a plurality of pulses may include a single cycle of a signal having a selected frequency. The frequency spectrum of such a pulse will be a signal centered at the selected frequency, having a bandwidth. Accordingly, pulse transmitter 212 may be configured to generate a plurality of voltage pulses centered at a plurality of frequencies. For example, the plurality of center frequencies for pulses provided by pulse transmitter 212 may include different frequencies, such as baseband frequencies and their harmonics. Thus, pulse transmitter circuit 212 has a transmission band which may include multiple center frequencies for a plurality of pulses provided to a transducer assembly.

In some embodiments, data 224 includes electrical signals received from catheter 102 and amplified by receive amplifier 214. The electrical signals in data 224 may be voltage signals. According to some embodiments, data 224 is an analog signal associated to an ultrasonic echo from a tissue structure around the transducer assembly. Analog-to-digital converter (ADC) 216 converts amplified electrical signal 224 into a digital signal. In some embodiments, the digital signal from ADC 216 is further processed by a reconstruction circuit 250. In some embodiments, data 224 includes voltage signals produced by the transducer assembly upon receiving an ultrasound echo signal from a tissue structure. The tissue structure may be surrounding a distal end of a catheter that includes the transducer assembly (e.g., distal end 120, cf. FIG. 1). The voltage signal in data 224 may include tissue responses at a plurality of frequencies, forming a reception band. Thus, receive amplifier 214 may include filters that produce a bandwidth including the reception band. Accordingly, in some embodiments the filtering of incoming data 224 and outgoing control signal 223 may be performed by ASIC 144 at distal end portion 120 of catheter 102 (cf. FIG. 1B).

Reconstruction circuit 250 may perform operations on the digitized, amplified data 224 such as data smoothing, averaging, noise filtering, and data interpolation. Thus, in some embodiments reconstruction circuit 250 may prepare the data provided by transducer assembly 122 for an image rendition of the tissue surrounding distal end 120 of catheter 102. The reconstructed digital data is transferred out of PIM 104 to IVUS control system 106 by a communication protocol circuit 218.

In some embodiments, a clock and timing circuit 200 provides a digitizing signal 226 to ADC 216, and transmitter timing signal 222 to pulse transmitter 212. According to some embodiments, clock and timing circuit 200 provides transmitter timing signal 222 and digitizing signal 226 using a common stable system clock. Some embodiments may include a phase-locked loop circuit in clock and timing circuit 200 to synchronize transmitter timing signal 222 and digitizing signal 226. In some embodiments transmitter timing signal 222 and digitizing signal 226 have the same phase, or their relative phase is fixed in time to within the resolution of clock and timing circuit 200.

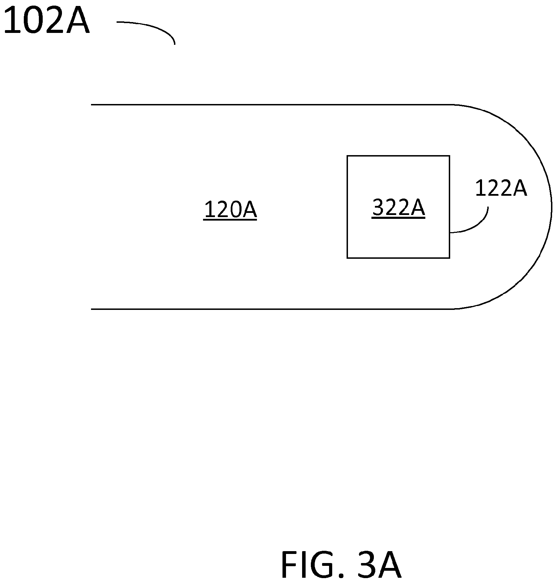

FIG. 3A is a partial illustration of a distal end 120A of a catheter 102A for multi-frequency imaging. Distal end 120A includes transducer assembly 122A. Accordingly, transducer assembly 122A includes a single piezo-electric component 322A. Piezo-electric component 322A may have a response band that includes the transmission band of a pulse transmitter 212 and the reception band of receive amplifier 214. The response band of piezo-electric component 322A is determined by the material forming the component, and the geometry of the component. The response band of a transducer element may be measured by the voltage amplitude produced when an acoustic wave of a certain frequency impinges on the transducer. It may be desirable to have a broad response bandwidth for piezo-electric component 322A. For example, a response band from about 5 MHz to about 135 MHz may be achievable using polymer-based transducer assemblies. The polymer used in transducer assembly 322A may be a ferroelectric polymer such as polyvinylidene fluoride (PVDF). A polymer used in transducer assembly 322A may include PVDF-co-trifluoroethylene (PVDF-TrFE) as a piezo-electric material. Alternatively, polymers such as PVDF-CTFE or PVDF-CFE may be used.

Thus, a pulse transmitter circuit 212 provides a plurality of pulses within a transmission band to a single piezo-electric component 322A. Likewise, a single piezo-electric component 322A may receive an ultrasound echo within a reception band from a tissue structure. The tissue structure may be a blood vessel wall surrounding distal end 120A of catheter 102.

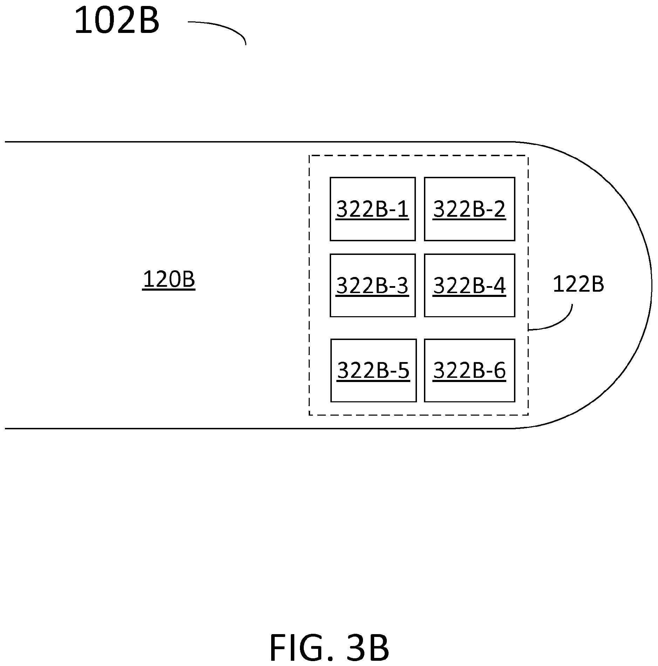

FIG. 3B is a partial illustration of a distal end 120B of a catheter 102B for multi-frequency imaging, according to some embodiments. Distal end 120B includes transducer assembly 122B. Transducer assembly 122B includes a plurality of piezo-electric components exemplified by 322B-1, 322B-2, 322B-3, 322B-4, 322B-5, and 322B-6 (collectively referred to hereinafter as 'piezo-electric components 322B'). One of ordinary skill will recognize that the specific number of piezo-electric components 322B in assembly 122B is arbitrary and not limiting. Furthermore, the specific shape and arrangement of components 322B is also not limiting, depending only in the specific application of a multi-frequency imaging catheter 102.

In some embodiments, a first selected group of components 322B is used for transmitting ultrasound pulses, and a second selected group of components 322B is used for receiving ultrasound echoes. The first selected group of components 322B is different from the second selected group of components 322B. Therefore, in some embodiments each of the components 322B-1 through 322B-6 may have a narrow response band, tuned to the specific function of the component (e.g., transmission, reception, or both). For example, component 322B-1 may have a response band including a portion of the transmission band. Likewise, component 322B-2 may have a response band including a portion of the reception band. Accordingly, a first selected group of ultrasound pulses provided to the first selected group of components 322B may be centered at a first selected group of frequencies. The first selected group of frequencies may include ultrasound frequencies such as 20 MHz, 40 MHz, and 80 MHz. A receive amplifier in a processor circuit for a PIM module coupled to transducer 122B has filters with bandwidths selected according to the response band of the second selected group of components 322B. Thus, radio-frequency (RF) filters in a response amplifier inside a PIM may be centered at a second selected group of frequencies such as 20 MHz, 40 MHz, and 80 MHz. In some embodiments, RF filters may be included in ASIC 144 at distal end portion 120 of catheter 102, to filter the echo signal in a selected response band.

Accordingly, the second selected group of frequencies includes harmonic combinations of the first selected group of frequencies. A harmonic combination may include integer multiples of a frequency in the first selected group of frequencies. In some embodiments, a harmonic combination may include a sum of frequencies from the first selected group of frequencies. For example, when a first selected group of frequencies includes 20 MHz, 40 MHz, and 80 MHz, a second selected group of frequencies may include 40 MHz (= 2 × 20 MHz), 80 MHz (= 2 × 40 MHz), 60 MHz (= 20 MHz + 40 MHz), and even 120 MHz (= 40 MHz + 80 MHz).

Thus, according to some embodiments, transducer assembly 122B may provide a wide response band by using multiple transducer components 322B. Each of transducer components 322B may have a narrow response band covering a portion of a transmission band or a portion of a reception band, centered at a selected frequency. Such a configuration may be desirable since piezo-electric components with a narrow response band may provide high response efficiency.

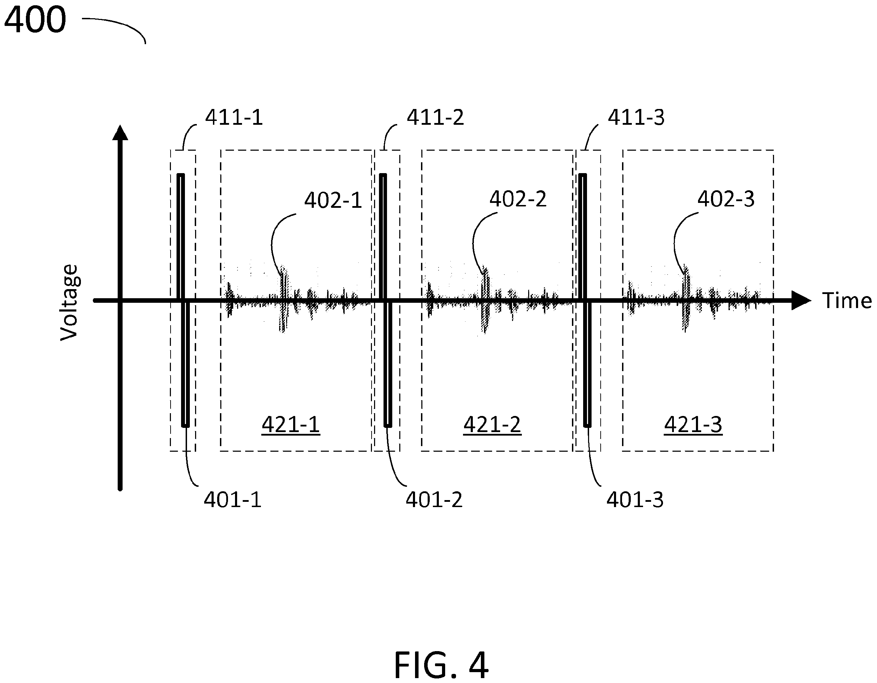

FIG. 4 is a partial schematic illustration of a transducer voltage 400. Transducer voltage 400 includes voltage values across a transducer component in a transducer assembly, as a function of time (e.g., transducer assemblies 122A, 122B in FIGS. 3A, 3B above). Transducer voltage 400 includes ultrasound pulse transmit portions 411-1, 411-2, and 411-3, collectively referred to hereinafter as 'transmission portions 411.' Likewise, transducer voltage 400 includes ultrasound echo reception portions 421-1, 421-2, and 421-3, collectively referred to hereinafter as 'reception portions 421.' While FIG. 4 illustrates three transmission portions 411 and three reception portions 421, one of ordinary skill will recognize that there is nothing limiting in the number of transmission portions 411, and reception portions 421 that may be used. The number of transmission portions 411 may be different from the number of reception portions 421. FIG. 4 illustrates each one of transmission portions 411 followed by a reception portion 421. It will be recognized by those with ordinary skill that one or more transmission portions 411 may be provided in sequence, before a reception portion 421 follows. Likewise, a plurality of reception portions 421 may be provided in sequence, after a transmission portion 411.

Transmission portions 411 include pulses 401-1, 401-2, and 401-3 provided by a pulse transmitter circuit (e.g., pulse transmitter 212, cf. FIG. 2) to the transducer assembly. Pulses 401-1 through 401-3 are collectively referred to hereinafter as pulses 401. Reception portions 421 include signals 402-1, 402-2, and 402-3 provided by the transducer assembly to an amplifier circuit (e.g., receive amplifier 214, cf. FIG. 2). Signals 402-1, 402-2, and 402-3 are referred to hereinafter as ultrasound echo signals 402. Accordingly, ultrasound echo signals 402 may be tissue responses to pulses 401. Moreover, depending on the frequency component of pulses 401, ultrasound echo signals 402 may proceed from different portions of a tissue structure surrounding the transducer assembly. Thus, ultrasound echo signal 402-3 corresponding to a pulse 401-3 having a high center frequency may proceed from a shallow region of the tissue structure. For example, a signal 402 corresponding to a pulse 401 centered at about 80 MHz may proceed from an area of a blood vessel wall proximate to the lumen of the vessel.

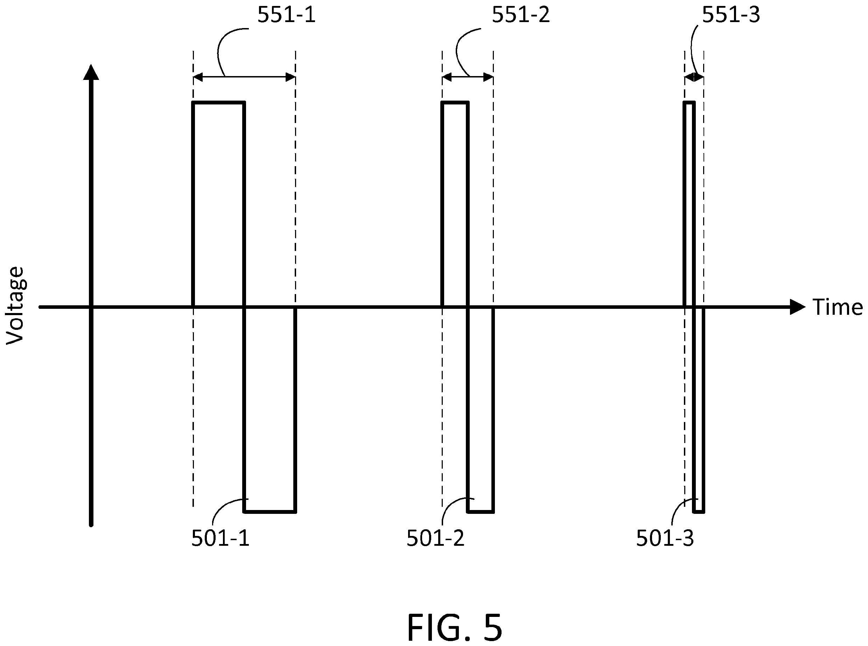

FIG. 5 is a partial schematic illustration of multi-frequency voltage pulses for a transducer component. FIG. 5 illustrates voltage pulses 501-1, 501-2, and 501-3, collectively referred hereinafter as voltage pulses 501. Consistent with FIG. 5, each of pulses 501 includes a cycle having a period 551-1, 551-2, and 551-3, collectively referred hereinafter as period values 551. Accordingly, period 551-1 may be different from period 551-2, thus corresponding to a pulse 501-1 centered at a lower frequency than the center frequency of pulse 501-2. For example, if period 551-1 is double the length of period 551-2, the center frequency of pulse 501-2 will be at double the frequency of the center of pulse 501-1. Likewise, period 551-3 may be even shorter than period 551-1, corresponding to an even higher center frequency for pulse 501-3. One of ordinary skill will recognize that pulses 501 may include any one of a plurality of period values 551.

In embodiments consistent with the present disclosure it is desirable that the transducer assembly receiving pulses 501 has a response bandwidth including the center frequencies of each of pulses 501. In some embodiments using a plurality of transducer components (e.g., components 322B, FIG. 3B), each of pulses 501 may be directed to a different transducer component optimized to operate at a specific center frequency. For example, transducer component 322B-1 may be designed to operate with maximum efficiency at the center frequency corresponding to period 551-1. One of ordinary skill will recognize that there is no limitation as to the number of different periods 551 that may be provided. Also, the specific center frequency associated to a given period 551 may be chosen according to a specific application or need. For example, a period 551 may correspond to a frequency of 20 MHz, 40 MHz, 80 MHz, or more. In some embodiments, one of periods 551 may include a frequency of 10 MHz, or less.

In addition to obtaining information from different portions of a tissue structure using multi-frequency pulses as in FIG. 5, images having different axial resolution may be obtained. For example, an image generated with ultrasound echo signal from pulse 501-1 may have longer penetration depth within the tissue, and lower axial resolution, compared to an image generated from pulse 501-2. An image obtained from pulse 501-3 may provide higher axial resolution at a lower penetration depth.

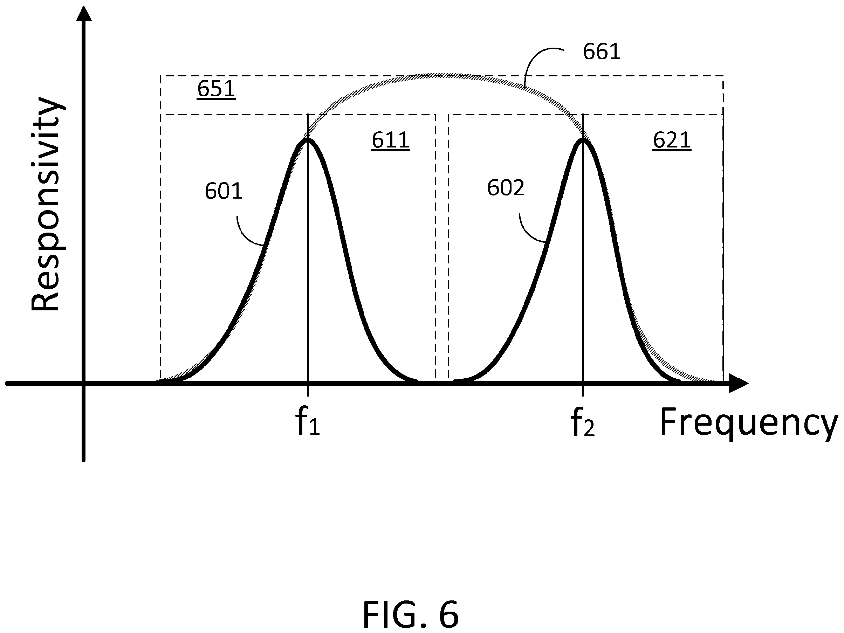

FIG. 6 is a partial schematic illustration of a transmission band 611, a reception band 621, and a response band 651 in a multi-frequency IVUS imaging system, according to some embodiments. In FIG. 6, response band 651 is a broad band including transmission band 611 and reception band 621. Transmission band 611 includes a transmission curve 601 centered at a first frequency f₁, and reception band 621 includes a reception curve 602 centered at a second frequency, f₂. According to some embodiments, the second frequency may be a second harmonic of the first frequency (f₂ = 2×f₁). An embodiment such as illustrated in FIG. 6 may correspond to the spectral configuration of a multi-frequency IVUS imaging system using a single transducer (e.g., transducer 122A, cf. FIG. 3A). In some embodiments reception band 621 may lay outside of response band 651 of the transducer providing transmission band 611. Such embodiments may correspond to a multi-frequency IVUS imaging system using a plurality of transducer components (e.g., transducer 122B, FIG. 3B). For example, transmission band 611 and response band 661 may correspond to transducer component 322B-1. Further, reception band 621 from component 322B-2 may be outside of response band 661.

A multi-frequency IVUS imaging system as disclosed herein may include a PIM coupled to a catheter having a transducer assembly with a transmission band, a reception band, and a response band as illustrated in FIG. 6 (e.g., system 100, PIM 104, catheter 102, and transducer assembly 122, FIG. 1). The PIM may include a processor circuit to provide a pulse signal to the transducer assembly and receive an ultrasound echo from the signal (e.g., processor circuit 154). The processor circuit may include a pulse transmitter circuit having a bandwidth such as transmission band 611 (e.g., pulse transmitter 212). The processor circuit may also include a receive amplifier having a bandwidth as reception band 621 (e.g., receive amplifier 214). The bandwidth of the pulse transmission circuit and the receive amplifier circuit may be adjusted using RF circuit filtering techniques. In some embodiments RF circuit filters may be included in ASIC 144, at distal end portion 120 of catheter 102.

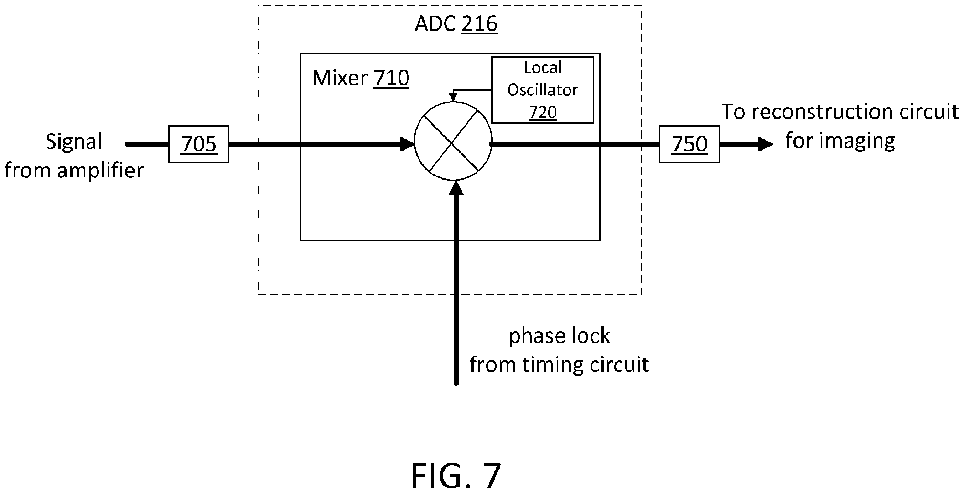

FIG. 7 is a partial schematic illustration of a signal processing strategy for selecting harmonic components 750 of a signal 705, according to some embodiments. In some embodiments, an ADC circuit includes a frequency mixer 710 (e.g., ADC 216, FIG. 2). Mixer 710 combines signal 705 from an amplifier circuit with a local oscillator signal at a harmonic frequency 720. In some embodiments, the harmonic frequency of the local oscillator is an integer multiple of a center frequency included in a pulse signal for a transducer assembly (e.g., any one of pulses 501, cf. FIG. 5). For example, if a center frequency in a pulse signal is 'f, the harmonic frequency 720 of the local oscillator in mixer 710 may be '2 × f'. Frequency mixer 710 may include a feedback circuit using a phase-lock signal provided by a timing circuit (e.g., clock and timing circuit 200, FIG. 2). For example, in some embodiments the phase lock signal may be included in digitizing signal 226 (cf. FIG. 2). Harmonic component 750 of signal 705 may thus be transmitted to a reconstruction circuit for imaging (e.g., reconstruction circuit 250).

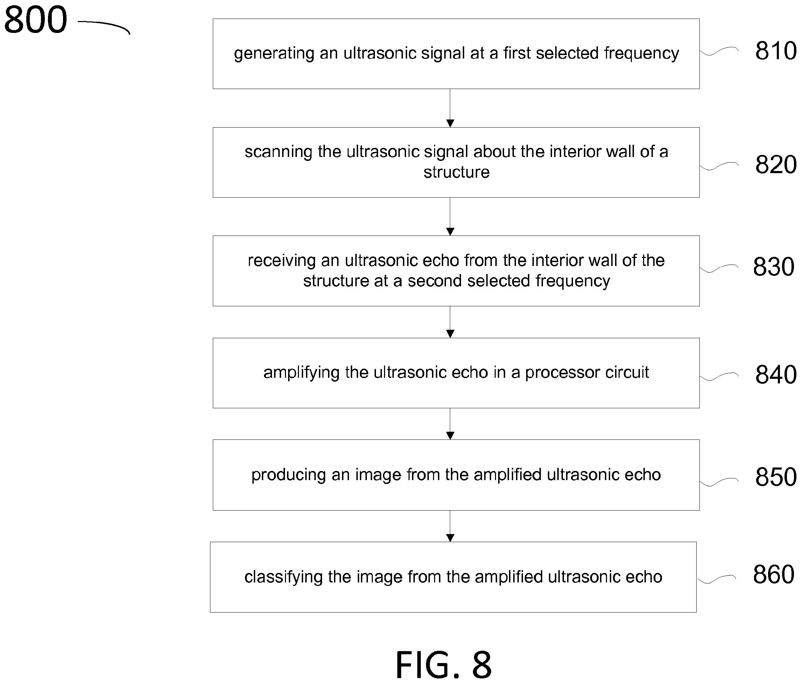

FIG. 8 is a flow chart illustrating steps in a method 800 for multi-frequency imaging. Method 800 may be performed by a control system (e.g., control system 106) using a processor circuit (e.g., processor circuit 156) and a memory circuit (e.g., memory circuit 157) and/or a PIM (e.g., PIM 104) using a processor circuit (e.g. processor circuit 154) and a memory circuit (e.g., memory circuit 155) based on scan data provided by a transducer assembly (e.g., transducer assembly 122 cf. FIG. 1). The transducer assembly may be in the distal end of a catheter positioned inside the lumen of a tissue structure (cf. catheter 102, FIG. 1). Steps in method 800 may be performed by the control system and steps in method 800 may be performed by the PIM. A reconstructed image plane in method 800 may be provided to a user in a display (e.g., display 108). The reconstructed image may be a 3-dimensional image (3D-image) including a plurality of cross-sectional planes of a tissue structure.

Step 810 includes generating an ultrasonic signal at a first selected frequency. The ultrasonic signal may be generated from a transducer assembly (e.g., transducer assembly 122, cf. FIG. 1) in the form of an ultrasound acoustic beam following a path. The ultrasound acoustic beam path may be focused, to produce a focal region of high intensity acoustic energy in a target portion of a tissue. Step 810 may include providing a voltage pulse to a transducer assembly 122A, B (cf. FIGS. 3A, B). Accordingly, a voltage pulse maybe provided by the PIM to transducer assemblies 122A, B for a pre-selected period of time. Furthermore, a voltage pulse for transducer assemblies 122A, B may be provided in a series of pulses such as pulses 401, or pulses 501 produced at a preselected center frequency (cf. FIGS. 4 and 5). Step 810 may include generating a plurality of pulses at a first plurality of center frequencies. The first plurality of center frequencies may include different center frequencies.

Step 820 includes scanning the ultrasonic signal in a predetermined pattern about the interior wall of a structure. Step 820 may include sweeping the ultrasonic signal continuously in a helicoid pattern about an interior wall of a blood vessel. Step 820 is accomplished by rotating the transducer or rotating a reflective surface which deflects the signal from the transducer within a catheter. The catheter may remain substantially stationary while the transducer is rotated and the acoustic signal is swept radially around the LA of the catheter. Step 820 includes a stationary transducer assembly having multiple components around a circumference (e.g., transducers 322B, cf. FIG. 3B). Thus, by sequentially altering the phase relationship between each of the components in the circumference, an ultrasonic beam may be scanned radially around the LA of the catheter.

Step 830 includes receiving an ultrasonic echo from the interior wall of the structure at a second selected frequency. Step 830 may be performed using the transducer assembly of step 810. Thus, upon receiving the ultrasonic echo from the interior wall of the structure, a deformation induced in a piezo-electric material in the transducer assembly may result in a voltage signal. The transducer assembly may be configured to couple the voltage signal out of a surrounding tissue structure into a processor circuit (e.g., processor circuit 154 in PIM 104).

Step 830 maybe performed during a period of time between two voltage pulses 401 generating acoustic signals according to step 810 (cf. FIG. 4). In step 810 a voltage signal travels from the processor circuit in the PIM to the transducer assembly along a catheter (e.g., catheter 102, FIG. 1) at a first selected frequency. In step 830 a voltage signal travels from the transducer assembly to the processor circuit in the PIM. The voltage signal may include the second selected frequency. The second selected frequency may be centered at a harmonic frequency of the first frequency. The second selected frequency includes the first plurality of selected frequencies of step 810. The second selected frequency may include a sum of two different frequencies from the first plurality of selected frequencies in step 810. The second selected frequency may include an integer multiple of either one of the frequencies from the first plurality of selected frequencies.

Step 840 includes amplifying the ultrasonic echo in the processor circuit. Step 840 may include filtering the ultrasonic echo signal using electronic filters having a band-pass including the second selected frequency. Accordingly, a portion of step 840 may be performed by an ASIC circuit at a distal end of the catheter (e.g., ASIC 144 in FIG. 1B). Step 850 includes producing an image from the ultrasonic echo. Step 850 may be performed partially by the PIM. For example, step 850 may be partially performed by a reconstruction circuit (e.g., reconstruction circuit 250, FIG. 2). Step 850 includes producing a 2-dimensional image (2D-image) of a cross section of the blood vessel wall. The cross-section may be substantially parallel to an XY-plane perpendicular to a LA oriented along the blood vessel and the catheter direction (cf. FIG. 1). Step 850 includes producing a 3-dimensional image (3D-image) of the blood vessel wall from a plurality of 2D-images.

Step 860 includes classifying the image from the amplified ultrasonic echo. Step 860 may be performed by the processor circuit and the memory circuit in the control system. Step 860 is performed by processor circuit 156 executing commands, retrieving and storing data, the commands and the data being stored in memory circuit 157. The commands executed by processor circuit 156 in control system 106 maybe included in an image characterization code stored in memory circuit 157.

The image characterization code may render a characterized tissue component map. The image characterization application may perform a spectral analysis of ultrasound echo information for a vessel cross-section. Thus, different plaque components may be determined and distinguished in the characterized tissue component map. For example, the characterization application mayuse a classification criterion including a rule based upon a location of a confluence of necrotic core within the vessel cross-section in relation to a border between the lumen and a plaque. A classification criterion may include a rule, based upon a location, in relation to a lumen-plaque border, of confluent necrotic core within the vessel cross-section; and rendering, in response to the classification, a plaque classification associated with the vessel cross-section. For example, a classification criterion may use the thickness of fibrous cap to determine the vulnerability of a plaque, and the likelihood of plaque rupture and thrombosis. Image characterization applications as used in step 860 may be as disclosed in US PAT. No. 7,627,156 entitled "Automated lesion analysis based upon automatic plaque characterization according to a classification criterion," US PAT. No. 7,175,597 entitled "Non-Invasive Tissue Characterization System and Method," and US PAT. No. 6,200,268 entitled "Vascular Plaque Characterization,".

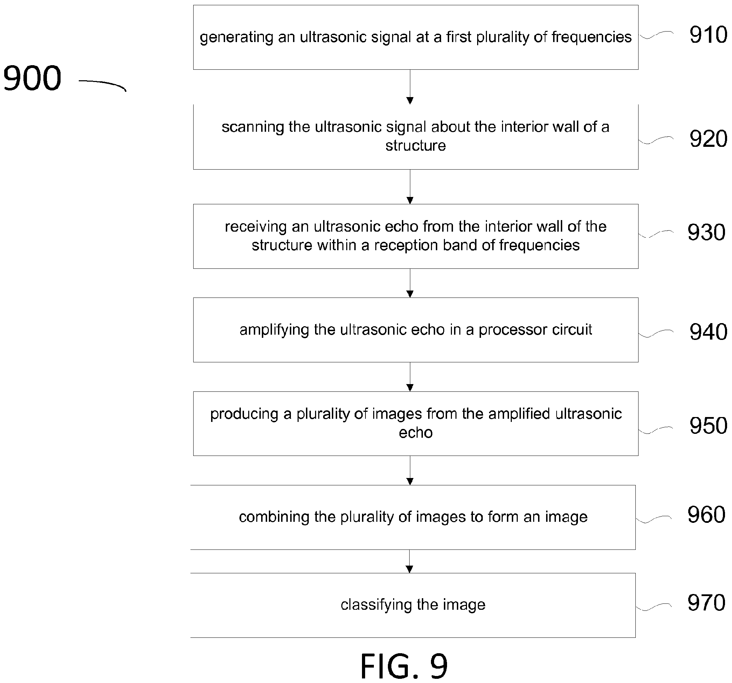

FIG. 9 is a flow chart illustrating steps in a method 900 for multi-frequency imaging. Method 900 may be performed partially by system 100. Method 900 may be performed by a control system (e.g., control system 106) using a processor circuit (e.g., processor circuit 156) and a memory circuit (e.g., memory circuit 157) and/or a PIM (e.g., PIM 104) using a processor circuit (e.g. processor circuit 154) and a memory circuit (e.g., memory circuit 155) based on scan data provided by a transducer assembly (e.g., transducer assembly 122 cf. FIG. 1). The transducer assembly may be positioned in the distal end of a catheter positioned inside the lumen of a tissue structure (cf. catheter 102, FIG. 1). Steps in method 900 may be performed by the control system and steps in method 900 may be performed by the PIM.

A reconstructed image plane in method 900 may be provided to an external operator in display 108. Thus, the external operator makes a decision of whether to excise a portion of the stenosed segment of the blood vessel using a recanalization tool, based on the reconstructed image plane on display 108. The recanalization tool maybe a physical instrument having a sharp end. The recanalization tool may be a laser beam susceptible of being directed to a point in the blood vessel and ablate a tissue portion. The recanalization tool may include an abrasive surface that may be rubbed against the target tissue.

Step 910 includes generating an ultrasonic signal at a first plurality of frequencies. Step 910 may further include positioning catheter 102 with its LA substantially aligned with the blood vessel, inside the lumen portion of the blood vessel. Step 920 includes scanning the ultrasonic signal in a predetermined pattern about the interior wall of a structure. The interior wall of a structure is the interior portion of a blood vessel wall including a region of stenosis. The region of stenosis may include a plaque having a calcified portion, a lipid pool, and a necrotic core adjacent to the lipid pool.

Step 930 includes receiving an ultrasonic echo from the interior wall of the structure, within a reception band of frequencies. A reception band of frequencies in step 930 may include the first plurality of frequencies. The reception band of frequencies may include harmonic combinations of the first plurality of frequencies. Harmonic combinations of the first plurality of frequencies may include integer multiples of either of the frequencies in the first plurality of frequencies. Furthermore, harmonic combinations of the first plurality of frequencies may include sums of any number of frequencies in the first plurality of frequencies. Step 940 includes amplifying the ultrasonic echo in a processor circuit. Step 940 may be as step 840 described in detail above, in relation to method 800. Step 950 includes producing a plurality of images from the amplified ultrasonic echo. Accordingly, step 950 may include producing an image for each of the frequencies within the reception band of frequencies in step 930. Producing each image in step 950 maybe as described in detail above, with relation to step 850, in method 800.

Step 960 includes combining the plurality of images from step 950 to form an image. Step 950 may include combining a portion of a first image obtained with a high frequency ultrasound echo with a portion of a second image obtained with a low frequency ultrasound echo. Accordingly, the portion of the first image may be a shallower portion of the tissue structure, and the portion of the second image may be a deeper portion of the tissue structure. Furthermore, the portion of the second image may filter out blood speckle artifacts typically encountered in high frequency ultrasound signal, such as used for the first image. For example, a first portion of an image of a blood vessel obtained at 80 MHz may include the endothelium of the blood vessel, including the border between the blood vessel wall and the lumen. A second portion of an image of a blood vessel obtained at 20 MHz may include portions of necrotic tissue in a plaque, macrophage cells, and muscle cells deeper into the blood vessel wall.

Step 970 includes classifying the image obtained in step 960. Step 970 may be performed by processor circuit 156 and memory circuit 157 in control system 106. Step 970 is performed by processor circuit 156 executing commands, retrieving and storing data, the commands and the data being stored in memory circuit 157. For example, the commands executed by processor circuit 156 in control system 106 may be included in an image characterization code stored in memory circuit 157. The image characterization code may render a characterized tissue component map. The image characterization application is able to perform a spectral analysis of ultrasound echo information for a blood vessel cross-section. Thus, different plaque components may be determined and distinguished in the characterized tissue map. For example, the characterization application may use a classification criterion including a rule based upon a location of a confluence of necrotic core within the vessel cross-section in relation to a border between the lumen and a plaque.

A classification criterion may use the thickness of a fibrous cap to determine the vulnerability of a plaque, and the likelihood of plaque rupture and thrombosis. For example, a plaque may be classified as 'vulnerable' to rupture, based on the size, configuration, and nature of its components. A component of a plaque within a blood vessel may be a fibrous cap and a necrotic core. In some configurations a component of a plaque within a vessel may include fat cell tissue and macrophage cells. The nature of a component of a plaque within a vessel may include substances such as elastin, collagen and cholesterol. The viscoelastic properties of the substances and the configuration of the different components included in a plaque within a vessel provide a differentiated acoustic response to the ultrasonic signals in step 910. Thus, interaction of the blood vessel wall with ultrasonic signals in step 910 may produce an image that clearly differentiates the components of the plaque, their nature, and their configuration (size and shape).

Embodiments of the invention described above are exemplary only. One skilled in the art may recognize various alternative embodiments from those specifically disclosed. As such, the invention is limited only by the following claims.

## Claims

1. An intravascular ultrasound (IVUS) imaging system (100) for imaging a volume within a patient, the IVUS imaging system comprising:
an interface module (104) comprising a processing circuit (154) and an IVUS catheter (102) comprising a plurality of piezo-electric transducer components at the distal end, said plurality having a first transducer component for generating (810) an ultrasonic signal at a first selected frequency and a second transducer component for receiving (830) an ultrasonic echo (224) at a second selected frequency, wherein the second frequency is a harmonic of the first frequency;
wherein the second transducer component has a response band including a portion of a reception band;
wherein the IVUS catheter is configured to direct the ultrasonic signal on a spot in the volume within the patient and scan (820) the spot in a predetermined pattern about the wall of the volume within the patient;
wherein the second transducer component is configured to convert the received ultrasonic echo into a voltage signal including the second selected frequency;
wherein the interface module (104) comprises a receive amplifier (214) having filters with bandwidths producing said reception band selected according to the said response band and configured to amplify (840) the voltage signal with a processing circuit (154); and
wherein the IVUS imaging system is configured to provide (850) an image of the volume within the patient from the amplified voltage signal.

2. The IVUS imaging system of claim 1 wherein the IVUS catheter further comprises an Application Specific Integrated Circuit (ASIC) positioned proximate to the first transducer component and the second transducer component, and wherein generating the ultrasonic signal and receiving the ultrasonic echo are partially performed by the ASIC.

3. The IVUS imaging system of claim 1 or claim 2 wherein the first transducer component and the second transducer component comprise a polymeric transducer material.

4. The IVUS imaging system of any of claims 1 to 3 wherein the first transducer component comprises a first plurality of transducer components and the second transducer component comprises a second plurality of transducer components.

5. The IVUS imaging system of claim 4 wherein the first selected frequency comprises a first plurality of frequencies, each of the first plurality of frequencies being associated with each of the first plurality of transducer components.

6. The IVUS imaging system of claim 5 wherein the second selected frequency comprises a second plurality of frequencies, each of the second plurality of frequencies being associated with each of the second plurality of transducer components.

7. The IVUS imaging system of claim 6 wherein the IVUS imaging system is configured to provide (950) a plurality of images of the volume within the patient, the plurality of images comprising an image for each of the frequencies in the second plurality of frequencies.

8. The IVUS imaging system of claim 7 wherein the IVUS imaging system is configured to combine at least one volume image associated with a first frequency and a second volume image associated with a second frequency in the second plurality of frequencies to form a combined volume image.

9. The IVUS imaging system of claim 8 wherein the IVUS imaging system is configured to combine a shallow portion of the volume at a higher frequency from the first and second frequencies with a deep portion of the volume at a lower frequency from the first and second frequencies.

10. The IVUS imaging system of claim 9 wherein the IVUS imaging system is configured to filter out a blood speckle signal from the combined volume image.

11. The IVUS imaging system of any of claims 7 to 10 wherein the PIM is configured to extract a frequency component from the voltage using a frequency mixer circuit in the processing circuit.

12. The IVUS imaging system of claim 11 wherein the frequency component is a harmonic combination of any of the frequencies in the first plurality of frequencies.

## Patentansprüche

1. Intravaskuläres Ultraschall-, (IVUS), Abbildungssystem (100) zum Abbilden eines Volumens innerhalb eines Patienten, das IVUS-Abbildungssystem umfassend:
ein Schnittstellenmodul (104), umfassend einen Verarbeitungsschaltkreis (154) und einen IVUS-Katheter (102), umfassend eine Vielzahl von piezoelektrischen Wandlerkomponenten beim distalen Ende, wobei die Vielzahl eine erste Wandlerkomponente zum Erzeugen (810) eines Ultraschallsignals bei einer ersten ausgewählten Frequenz und eine zweite Wandlerkomponente zum Empfangen (830) eines Ultraschallechos (224) bei einer zweiten ausgewählten Frequenz hat, wobei die zweite Frequenz eine Oberwelle der ersten Frequenz ist;
wobei die zweite Wandlerkomponente ein Antwortband hat, das einen Abschnitt eines Empfangsbands enthält;
wobei der IVUS-Katheter konfiguriert ist, das Ultraschalsignal auf einen Punkt im Volumen innerhalb des Patienten zu richten und den Punkt in einem vorbestimmten Muster um die Wand des Volumens innerhalb des Patienten abzutasten (820);
wobei die zweite Wandlerkomponente konfiguriert ist, das empfangene Ultraschallecho in ein Spannungssignal umzuwandeln, das die zweite ausgewählte Frequenz enthält;
wobei das Schnittstellenmodul (104) einen Empfangsverstärker (214) mit Filtern mit Bandbreiten umfasst, die das Empfangsband erzeugen, das gemäß dem Antwortband ausgewählt ist und konfiguriert ist, das Spannungssignal mit einem Verarbeitungsschaltkreis (154) zu verstärken (840); und
wobei das IVUS-Abbildungssystem konfiguriert ist, ein Bild des Volumens innerhalb des Patienten aus dem verstärkten Spannungssignal bereitzustellen (850).

2. IVUS-Abbildungssystem nach Anspruch 1, wobei der IVUS-Katheter weiter einen anwendungsspezifischen integrierten Schaltkreis (ASIC) umfasst, der nahe der ersten Wandlerkomponente und der zweiten Wandlerkomponente positioniert ist und wobei Erzeugen des Ultraschallsignals und Empfangen des Ultraschallechos teilweise durch den ASIC ausgeführt werden.

3. IVUS-Abbildungssystem nach Anspruch 1 oder Anspruch 2, wobei die erste Wandlerkomponente und die zweite Wandlerkomponente ein polymeres Wandlermaterial umfassen.

4. IVUS-Abbildungssystem nach einem der Ansprüche 1 bis 3, wobei die erste Wandlerkomponente eine erste Vielzahl von Wandlerkomponenten umfasst und die zweite Wandlerkomponente eine zweite Vielzahl von Wandlerkomponenten umfasst.

5. IVUS-Abbildungssystem nach Anspruch 4, wobei die erste ausgewählte Frequenz eine erste Vielzahl von Frequenzen umfasst, wobei jede der ersten Vielzahl von Frequenzen jeder der ersten Vielzahl von Wandlerkomponenten zugehörig ist.

6. IVUS-Abbildungssystem nach Anspruch 5, wobei die zweite ausgewählte Frequenz eine zweite Vielzahl von Frequenzen umfasst, wobei jede der zweiten Vielzahl von Frequenzen jeder der zweiten Vielzahl von Wandlerkomponenten zugehörig ist.

7. IVUS-Abbildungssystem nach Anspruch 6, wobei das IVUS-Abbildungssystem konfiguriert ist, eine Vielzahl von Bildern des Volumens innerhalb des Patienten bereitzustellen (950), wobei die Vielzahl von Bildern ein Bild für jede der Frequenzen in der zweiten Vielzahl von Frequenzen umfassen.

8. IVUS-Abbildungssystem nach Anspruch 7, wobei das IVUS-Abbildungssystem konfiguriert ist, mindestens ein Volumenbild, das einer ersten Frequenz zugehörig ist, und ein zweites Volumenbild, das einer zweiten Frequenz in der zweiten Vielzahl von Frequenzen zugehörig ist, zu kombinieren, um ein kombiniertes Volumenbild zu bilden.

9. IVUS-Abbildungssystem nach Anspruch 8, wobei das IVUS-Abbildungssystem konfiguriert ist, einen flachen Abschnitt des Volumens bei einer höheren Frequenz aus den ersten und zweiten Frequenzen mit einem tiefen Abschnitt des Volumens bei einer niedrigeren Frequenz aus den ersten und zweiten Frequenzen zu kombinieren.

10. IVUS-Abbildungssystem nach Anspruch 9, wobei das IVUS-Abbildungssystem konfiguriert ist, ein Blut-Speckle-Signal aus dem kombinierten Volumenbild zu filtern.

11. IVUS-Abbildungssystem nach einem der Ansprüche 7 bis 10, wobei der PIM konfiguriert ist, eine Frequenzkomponente aus der Spannung unter Verwendung eines Frequenzmischerschaltkreises im Verarbeitungsschaltkreis zu extrahieren.

12. IVUS-Abbildungssystem nach Anspruch 11, wobei die Frequenzkomponente eine Oberwellenkombination von irgendwelchen der Frequenzen in der ersten Vielzahl von Frequenzen ist.

## Revendications

1. Système d'imagerie ultrasonique intravasculaire (IVUS) (100) pour imager un volume au sein d'un patient, le système d'imagerie IVUS comprenant :
un module d'interface (104) comprenant un circuit de traitement (154) et un cathéter IVUS (102) comprenant une pluralité de composants transducteurs piézoélectriques à l'extrémité distale, ladite pluralité ayant un premier composant transducteur pour générer (810) un signal ultrasonique à une première fréquence sélectionnée et un second composant transducteur pour recevoir (830) un écho ultrasonique (224) à une seconde fréquence sélectionnée, dans lequel la seconde fréquence est une harmonique de la première fréquence ;
dans lequel le second composant transducteur a une bande de réponse incluant une partie d'une bande de réception ;
dans lequel le cathéter IVUS est configuré pour diriger le signal ultrasonique sur une tache dans le volume au sein du patient et balayer (820) la tache selon un motif prédéterminé autour de la paroi du volume au sein du patient ;
dans lequel le second composant transducteur est configuré pour convertir l'écho ultrasonique reçu en un signal de tension incluant la seconde fréquence sélectionnée ;
dans lequel le module d'interface (104) comprend un amplificateur de réception (214) ayant des filtres avec des largeurs de bande produisant ladite bande de réception sélectionnée selon ladite bande de réponse et configuré pour amplifier (840) le signal de tension avec un circuit de traitement (154) ; et
dans lequel le système d'imagerie IVUS est configuré pour fournir (850) une image du volume au sein du patient à partir du signal de tension amplifié.

2. Système d'imagerie IVUS selon la revendication 1, dans lequel le cathéter IVUS comprend en outre un circuit intégré spécifique à l'application (ASIC) positionné à proximité du premier composant transducteur et du second composant transducteur et dans lequel la génération du signal ultrasonique et la réception de l'écho ultrasonique sont en partie effectuées par l'ASIC.

3. Système d'imagerie IVUS selon la revendication 1 ou la revendication 2, dans lequel le premier composant transducteur et le second composant transducteur comprennent un matériau polymère pour transducteur.

4. Système d'imagerie IVUS selon l'une quelconque des revendications 1 à 3, dans lequel le premier composant transducteur comprend une première pluralité de composants transducteurs et le second composant transducteur comprend une seconde pluralité de composants transducteurs.

5. Système d'imagerie IVUS selon la revendication 4, dans lequel la première fréquence sélectionnée comprend une première pluralité de fréquences, chacune de la première pluralité de fréquences étant associée à chacun de la première pluralité de composants transducteurs.

6. Système d'imagerie IVUS selon la revendication 5, dans lequel la seconde fréquence sélectionnée comprend une seconde pluralité de fréquences, chacune de la seconde pluralité de fréquences étant associée à chacun de la seconde pluralité de composants transducteurs.

7. Système d'imagerie IVUS selon la revendication 6, dans lequel le système d'imagerie IVUS est configuré pour fournir (950) une pluralité d'images du volume au sein du patient, la pluralité d'images comprenant une image pour chacune des fréquences de la seconde pluralité de fréquences.

8. Système d'imagerie IVUS selon la revendication 7, dans lequel le système d'imagerie IVUS est configuré pour combiner au moins une première image volumique associée à une première fréquence et une seconde image volumique associée à une seconde fréquence de la seconde pluralité de fréquences pour former une image volumique combinée.

9. Système d'imagerie IVUS selon la revendication 8, dans lequel le système d'imagerie IVUS est configuré pour combiner une partie peu profonde du volume à une fréquence plus élevée parmi les premières et secondes fréquences avec une partie profonde du volume à une fréquence plus faible parmi les premières et secondes fréquences.

10. Système d'imagerie IVUS selon la revendication 9, dans lequel le système d'imagerie IVUS est configuré pour séparer par filtration un signal de mouchetures de sang de l'image volumique combinée.

11. Système d'imagerie IVUS selon l'une quelconque des revendications 7 à 10, dans lequel le PIM est configuré pour extraire une composante de fréquence de la tension en utilisant un circuit mélangeur de fréquences dans le circuit de traitement.

12. Système d'imagerie IVUS selon la revendication 11, dans lequel la composante de fréquence est une combinaison harmonique de certaines quelconques des fréquences de la première pluralité de fréquences.
